(19) Europäisches Patentamt
European Patent Office
Office européen des brevets

(11) Veröffentlichungsnummer: **0 199 206 A2**

(12) **EUROPÄISCHE PATENTANMELDUNG**

(21) Anmeldenummer: **86104952.6**

(22) Anmeldetag: **10.04.86**

(51) Int. Cl.⁴: **C07D 233/90**, C07D 233/64, C07D 413/04, C07D 403/06, C07D 403/04, C07D 403/12, A01N 43/50, A01N 43/653, A01N 43/713, A01N 43/76, A01N 43/82

(30) Priorität: **19.04.85 DE 3514116**

(43) Veröffentlichungstag der Anmeldung: **29.10.86 Patentblatt 86/44**

(84) Benannte Vertragsstaaten: **AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Schmierer, Roland, Dr.**
**An der Weinleite 5a**
**D-8901 Todtenweis(DE)**
Erfinder: **Mildenberger, Hilmar, Dr.**
**Fasanenstrasse 24**
**D-6233 Kelkheim (Taunus)(DE)**
Erfinder: **Handte, Reinhard, Dr.**
**Theilweg 23**
**D-8901 Gablingen(DE)**
Erfinder: **Bürstell, Helmut, Dr.**
**Am Hohlacker 65**
**D-6000 Frankfurt am Main 50(DE)**
Erfinder: **Bauer, Klaus, Dr.**
**Kolpingstrasse 7**
**D-6054 Rodgau(DE)**
Erfinder: **Bieringer, Hermann, Dr.**
**Eichenweg 26**
**D-6239 Eppstein/Taunus(DE)**
Erfinder: **Sachse, Burkhard, Dr.**
**An der Ziegelei 30**
**D-6233 Kelkheim (Taunus)(DE)**

(54) **Cyclohexyl- und cyclohexenylimidazolverbindungen, Verfahren zu ihrer Herstellung und ihre Verwendung als Pflanzenschutzmittel.**

(57) Die neuen Verbindungen der Formel I oder deren Salze

EP 0 199 206 A2

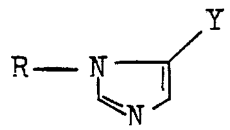

I

worin

R = einen (subst.) Cyclohexyl oder einen (subst.) Cyclohexenyl-rest; Y = Cyano, einen (subst.) (Di)-(Thio) Carbonsäure-(ester), -amid, -acetalrest, einen Keto-oder Oximrest oder einen heterocyclischen Rest bedeuten, besitzen vorteilhafte Wirkungen als Pflanzenwachstumregulatoren und weisen zudem günstige herbizide und fungizide Eigenschaften auf.

# Cyclohexyl-und Cyclohexenylimidazolverbindungen, Verfahren zu ihrer Herstellung und ihre Verwendung als Pflanzenschutzmittel

Es ist bekannt, daß 1-Phenylimdidazol-5-carbonsäurederivate, die in den Patentanmeldungen DE-A 32 17 094 und DE-A 34 44 918 beschrieben werden, pflanzenwachstumregulierende und teilweise herbizide Eigenschaften besitzen. Überraschenderweise wurde nun gefunden, daß 1-Cyclohexyl-und 1-Cyclohexenyl-imidazol-5-carbonsäurederivate gegenüber diesen bekannten Verbindungen vorteilhafte pflanzenwachstumsregulatorische, herbizide und fungizide Eigenschaften besitzen.

Die vorliegende Erfindung betrifft daher die neuen Verbindungen der Formel I

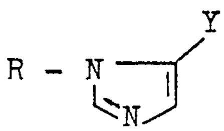

I

worin

R= einen Rest der Formeln

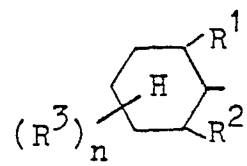

oder

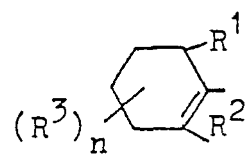

,

n= 0 bis 6,

Y= $-CN$, $-COOR^4$, $-COSR^5$, 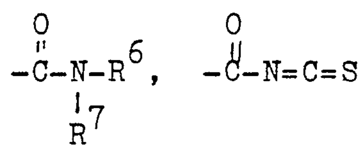

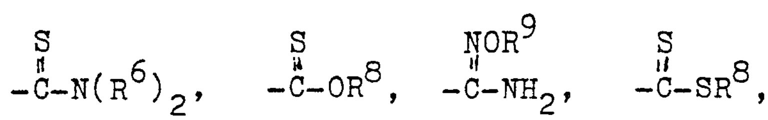

$-CH_2-OR^{10}$,

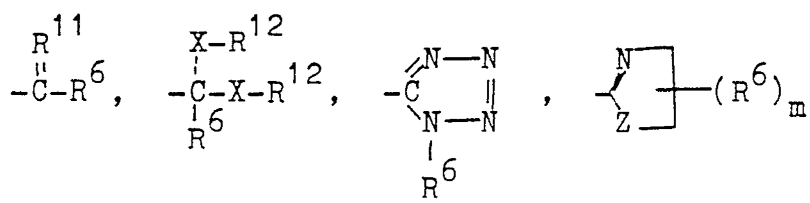

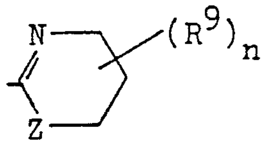,
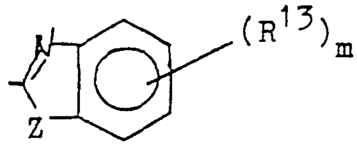,

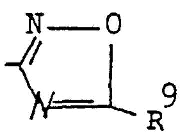 oder 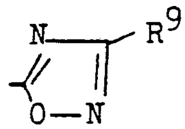,

m = 1 bis 4,

Z = O, S oder N-$R^6$,

X = O oder S,

$R^1$, $R^2$ unabhängig voneinander $(C_1-C_4)$Alkyl oder Wasserstoff,

$R^3$ jeweils unabhängig voneinander $(C_1-C_4)$-Alkyl, $(C_1-C_4)$Alkoxy, $(C_1-C_4)$Hydroxyalkyl, $(C_1-C_4)$-Alkoxy$(C_1-C_4)$alkyl, oder Halogen,

$R^4$ Wasserstoff, $(C_1-C_{12})$Alkyl, $(C_1-C_{12})$-Alkyl, das einbis dreifach substituiert ist durch Hydroxy, Halogen, $(C_1-C_6)$Alkoxy, $(C_1-C_4)$Alkoxy-$(C_1-C_4)$alkoxy, $(C_1-C_4)$Alkylthio, $(C_1-C_4)$-Alkylsulfinyl, $(C_1-C_4)$Alkylsulfonyl, Mono-oder Di-$(C_1-C_4$-alkyl)amino, Cyano, Aminocarbonyl, $(C_1-C_4)$Alkanoyl, $(C_1-C_4$-Alkoxy)carbonyl, Cyclo$(C_3-C_7)$alkyl, Tri$(C_1-C_4)$alkyl)silyl, Benzyloxy, Benzyloxyethoxy, Phenyl oder Phenyl, das durch Halogen oder $(C_1-C_4)$Alkyl ein-oder mehrfach substituiert ist, oder Phenoxy, Phenylthio, die beide durch Halogen oder $(C_1-C_4)$Alkyl ein-oder mehrfach substituiert sein können, ferner durch Oxiranyl, Tetrahydrofuryl, Triazolyl, Pyridinyl, Imidazolyl, Carboxy, Carboxylat mit einem für die Landwirtschaft einsetzbaren Kation oder den Rest -O-N=C-$(CH_3)_2$;

$(C_3-C_6)$Alkenyl, halogeniertes $(C_3-C_6)$Alkenyl, unsubstituiertes oder durch Halogen oder -$(C_1-C_4)$Alkyl substituiertes Cyclo$(C_3-C_7)$alkyl, unsubstituiertes oder durch Halogen oder ($C_1-C_4$)Alkyl substituiertes Cyclo $(C_5-C_7)$-alkenyl, $(C_3-C_6)$Alkinyl, 1,2-Epoxy-prop-3-yl, Phenyl oder Phenyl, das ein oder zweifach durch Halogen, Nitro, Cyano, $(C_1-C_4)$Alkyl, $(C_1-C_4$-Alkoxy)carbonyl oder $(C_1-C_4)$Alkoxy substituiert ist,

$(C_1-C_4$-Alkyl)carbonyl, Phenylcarbonyl, wobei der Phenylring durch Halogen, Nitro, Cyano, $(C_1-C_4)$Alkyl substituiert sein kann,

einen Rest der Formeln 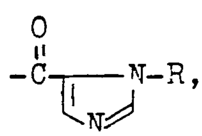,

$-N=CR^{14}R^{15}$, $-NR^6R^{16}$,

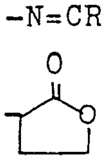, 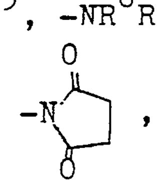, 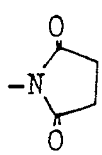 oder 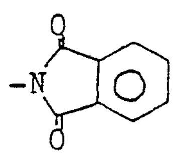;

$R^5$ $(C_1-C_{12})$Alkyl oder $(C_1-C_{12})$Alkyl, das bis zu zweifach durch $(C_1-C_4)$Alkoxyethoxy, Cyclo-$(C_3-C_6)$alkyl, Benzyloxy, Phenyl, Phenoxy, -$(C_1-C_4)$Alkylthio, $(C_1-C_4$-Alkoxy)carbonyl, Carboxy oder Carboxylat mit einem für die Landwirtschaft einsetzbares Kation, substituiert ist,

$R^6$ jeweils unabhängig voneinander Wasserstoff, $(C_1-C_6)$Alkyl, Phenyl oder $(C_3-C_6)$-Alkenyl,

$R^7$ Wasserstoff, $(C_1-C_{12})$Alkyl oder $(C_1-C_{12})$-

Alkyl, das bis zu zweifach, durch $(C_1-C_6)$-Alkoxy, $(C_1-C_4)$-Alkoxyethoxy, Hydroxy, Halogen, Cyclo$(C_3-C_6)$alkyl, Benzyloxy, Cyano, Aminocarbonyl, Carboxy, $(C_1-C_4$-Alkoxy)-carbonyl, Formyl, Phenyl oder Phenoxy substituiert ist, Phenyl oder Phenyl, das bis zu zweifach durch Halogen, Nitro, Cyano $(C_1-C_4)$Alkyl oder $(C_1-C_4)$Alkoxy substituiert ist; $(C_3-C_6)$-Alkenyl, $(C_3-C_6))$Cycloalkyl, einen Rest der Formeln $-NR^6R^{17}$, $-OR^9$, $-NH-CO-NH_2$, $-NH-CS-NH_2$ oder $-SO_2R^{18}$ oder

$R^6$ und $R^7$ gemeinsam mit dem Stickstoffatom an das sie gebunden sin, einen gesättigten oder ungesättigten gegebenenfalls benzokondensierten drei-bis siebengliedrigen Ring, der bis zu drei Heteroatome aus der Gruppe O, N und S enthält und der unsubstituiert oder durch $(C_1-C_4)$Alkyl oder Halogen substituiert ist und eine Carbonylgruppe enthalten kann,

$R^8$ H, $(C_1-C_6)$Alkyl oder Phenyl, oder im Falle R= $-CS-OR^8$ ein für die Landwirtschaft einsetzbares Kation,

$R^9$ jeweils unabhängig voneinander H, $(C_1-C_4)$Alkyl oder Benzyl;

$R^{10}$ jeweils unabhängig voneinander H, unsubstituiertes oder durch Phenyl, Halogenphenyl, Nitrophenyl, Cyanophenyl, $(C_1-C_4)$-Alkylphenyl oder $(C_1-C_4)$Alkoxyphenyl, Hydroxy, Cyano, $(C_1-C_4$-Alkoxy)carbonyl, $(C_1-C_4)$Alkylthio, $(C_1-C_4)$Alkoxy, Cyclo$(C_5-C_7)$alkyl oder Benzyloxy substituiertes $(C_1-C_{12})$alkyl;

Cyclo$(C_5-C_8)$alkyl, $(C_3-C_6)$Alkenyl, Halogen$(C_3-C_6)$alkenyl, $(C_3-C_6)$Alkinyl, Cyclo$(C_5-C_6)$-alkenyl, $(C_1-C_6$-Alkyl)carbonyl, Halogen$(C_1-C_6$-alkyl)carbonyl, $[(C_1-C_6$-Alkyl)amino]carbonyl, Benzoyl, Halogenbenzoyl oder Methylbenzoyl;

$R^{11}$ Sauerstoff (einschließlich der Bisufitaddukte) oder der Rest $=N-O-R^{10}$,

$R^{12}$ jeweils unabhängig voneinander unsubstituiertes oder durch Phenyl, Cyclo$(C_5-C_7)$-alkyl, $(C_1-C_4)$Alkoxy, $(C_1-C_4)$Alkylthio oder Halogen substituiertes $(C_1-C_6)$Alkyl;

oder beide Rest $R^{12}$ gemeinsam mit X und dem Kohlenstoffatom an das sie gebunden sind, einen unsubstituierten oder durch $(C_1-C_4)$Alkyl, Hydroxy$(C_1-C_4)$alkyl, Halogen$(C_1-C_4)$alkyl oder Phenyl substituierten 5-oder 6-gliedrigen gesättigten heterocyclischen Ring,

$R^{13}$ jeweils unabhängig voneinander H, Halogen, $(C_1-C_4)$Alkyl, Nitro oder Cyano,

$R^{14}$, $R^{15}$ unabhängig voneinander H, unsubstituiertes oder durch $(C_1-C_4)$Alkoxy, Triazolyl oder Imidazolyl substituiertes $(C_1-C_6)$Alkyl, Cyclo$(C_3-C_6)$alkyl, $(C_3-C_6)$Alkenyl, Phenyl oder Benzyl; oder

$R^{14}$ und $R^{15}$ gemeinsam mit dem Kohlenstoffatom, an das sie gebunden sind, ein unsubstituiertes oder durch Methyl oder Halogen substituiertes Cyclo-$(C_5-C_7)$alklyl,

$R^{16}$ $(C_1-C_4)$Alkyl, Phenyl, $(C_1-C_6$-Alkyl)-carbonyl, Benzyl, Benzoyl, Halogenbenzyl, Halogenbenzoyl oder Methylbenzoyl,

$R^{17}$ H, $(C_1-C_4)$Alkyl, Formyl, $(C_1-C_6$-Alkyl)-carbonyl, Benzoyl, Halogenbenzoyl, Methylbenzoyl oder Trihalogenacetyl und

$R^{18}$ $(C_1-C_4)$Alkyl, Phenyl oder Methylphenyl bedeuten, sowie deren für landwirtschaftliche Zwecke verträglichen Salze und Quaternisierungsprodukte.

Die Salzbildung bzw. Quaternisierung erfolgt entweder an einer freien Carboxygruppe z.B. im Falle $R^4$ oder $R^8$ = H oder am basischen Stickstoffatom des Imidazolrings. Die Salzbildung oder Quaternisierung am Imidazolring ist nicht möglich, wenn $R^4$, $R^8$ ein Kation bedeutet oder $R^4$, $R^5$ eine Carboxylatgruppe enthält.

Die Erfindung umfaßt alle optischen Isomeren der Verbindungen der Formel I. Diese können auftreten, wenn sie asymmetrisch substituierte Cycloalkyl-oder Cycloalkenylringe enthalten. Die bei der Definition der allgemeinen Formel (I) vorkommenden Alkyl-, Alkenyl-und Alkinylreste können sowohl geradkettig als auch verzweigt sein; unter Halogen ist F, Cl, Br oder J, insbesondere F, Cl oder Br zu verstehen.

Halogeniertes $(C_3-C_6)$Alkenyl enthält insbesondere 1 bis 3 Chlor oder Fluoratome.

Halogenphenyl, Halogenbenzyl oder Halogenbenzoyl enthalten insbesondere 1 bis 3 Fluor, Chlor oder Bromatome.

Unter Trihalogenacetyl ist insbesondere Trichlor-und Trifluoracetyl zu verstehen.

Ferner sind bevorzugt solche Verbindungen der Formel I und ihre Salze, worin R= einen Cyclohexyl-Rest der Formel

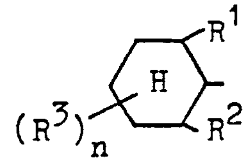,

n= 0 oder 1,

Y= -CN, -COOR$^4$, -COSR$^5$, 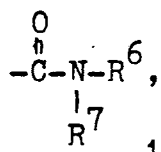, 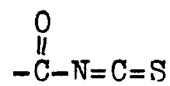,

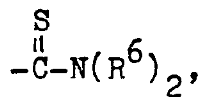, $-CH_2-OR^{10}$, 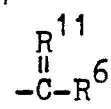, 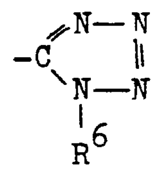

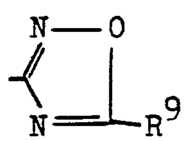 oder 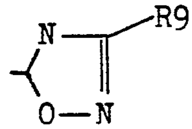,

R$^1$, R$^2$ unabhängig voneinander ($C_1$-$C_4$)Alkyl; R$^3$ jeweils ($C_1$-$C_4$)Alkyl, oder Halogen; R$^4$ Wasserstoff, -($C_1$-$C_6$)-Alkyl, ($C_1$-$C_6$)-Alkyl, das ein-bis dreifach durch Hydroxy, Halogen, ($C_1$-$C_6$)Alkoxy, ($C_1$-$C_6$)-Alkylthio, ($C_3$-$C_7$)Cycloalkyl oder Phenyl substituiert ist, ($C_3$-$C_6$))Alkenyl, halogeniertes ($C_3$-$C_6$)Alkenyl, Cyclo($C_3$-$C_6$)alkyl, ($C_3$-$C_6$)Alkinyl, Phenyl einen Rest der Formeln

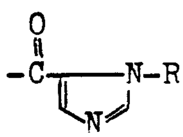 oder

-N=CR$^{14}$R$^{15}$; R$^5$ ($C_1$-$C_4$)Alkyl; R$^6$ Wasserstoff, ($C_1$-$C_4$)Alkyl, Phenyl oder Allyl; R$^7$ Wasserstoff, ($C_1$-$C_{12}$)-Alkyl oder ($C_1$-$C_{12}$)Alkyl, das bis zu zweifach durch ($C_1$-$C_6$)Alkoxy, Hydroxy, Halogen, Cyclo($C_3$-$C_6$)alkyl, Benzyloxy, Cyano, Aminocarbonyl, Carboxy, ($C_1$-$C_4$-Alkoxy)carbonyl, Formyl substituiert ist, Phenyl oder Phenyl, das bis zu zweifach durch Halogen, Nitro, Cyano, ($C_1$-$C_4$)Alkyl oder ($C_1$-$C_4$)Alkoxy substituiert ist; Allyl, ($C_3$-$C_6$)Cycloalkyl, einen Rest der Formeln -NR$^8$R$^{17}$, OR$^9$ oder

R$^6$ und R$^7$ bilden gemeinsam mit dem Stickstoffatom einen heterocyclischen Ring der Formeln

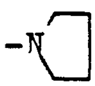, 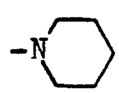, 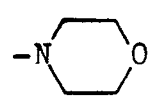, 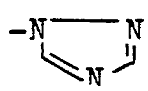 oder 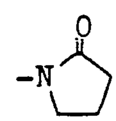,

$R^9$ H oder $(C_1$-$C_4)$Alkyl

$R^{10}$ Wasserstoff, $R^{11}$ Sauerstoff (einschließlich der Bisulfitaddukte) oder den Rest $=N$-$O$-$R^{10}$,

$R^{14}$, $R^{15}$ unsubstituiertes $(C_1$-$C_4)$Alkyl oder

$R^{14}$ und $R^{15}$ gemeinsam mit dem Kohlenstoffatom, an das sie gebunden sind, Cyclo-$(C_5$-$C_7)$alkyl und

$R^{17}$ H, $(C_1$-$C_4)$Alkyl oder Formyl bedeuten.

Für den Fall, daß in den aufgeführten Substituenten -zusätzlich zum Imidazolring -weitere basische Stickstoffatome auftreten, ist auch eine mehrfache Salzbildung oder Quaternisierung möglich.

Für die Salze geeignet sind alle anorganischen oder organischen Säuren, die aufgrund ihres pKs-Wertes zur Salzbildung befähigt sind, z.B. Halogenwasserstoffsäuren, Salpetersäure, Schwefelsäure, Phosphorsäure, Phosphonsäuren, Sulfonsäuren, Halogenessigsäuren oder Oxalsäure.

Als Quaternisierungsprodukte sind die Umsetzungsprodukte mit Alkyl-, Alkylthioalkyl-, Alkoxyalkyl-, insbesondere $(C_1$-$C_6)$Alkyl-und gegebenenfalls im Phenylrest substituierten, insbesondere halogenierten Phenacylhalogeniden zu verstehen. Die Herstellung der Quaternisierungsprodukte der Verbindungen der Formel I erfolgt nach allgemein üblichen Methoden.

Als Kationen für $R^4$, $R^8$ oder $R^5$, die für die Landwirtschaft einsetzbar sind kommen Metallkationen z.B. Alkali-oder Erdalkalikationen wie Na, K Mg oder organische Kationen wie organisches substituiertes Ammonium organisch substituiertes Phosphonium, Sulfonium oder Sulfoxonium oder andere Stickstoff-Kationen in Betracht.

Organisch substituiertes Ammonium bedeutet primäres, sekundäres, tertiäres, quartäres, aliphatisches, aromatisches oder heteroaromatisches Ammonium, das 1 bis drei N-Atome enthalten kann. Die Stickstoffatome des Amins können hierbei auch Teil eines cyclischen Systems sein. Als Beispiele für solche Ammoniumsalze seien genannt: Mono-, Di-, Tri-, Tetra[$(C_1$-$C_6)$Alkyl]ammonium wie Isopropylammonium, Butylammonium, Stearylammonium, Triethylammonium, Mono-, Di-, Tri-[$(C_1$-$C_4)$alkyl]ammonium oder Mono-, Di-, Tri-[$(C_1$-$C_6)$-alkanol]-ammonium wie Methoxyethylammonium, Methoxypropylammonium, Triethanolammonium, Tripropanolammonium, oder Ammoniumverbindungen mit gemischten Resten wie tert.-Butyldiethanolammonium, Triethylbenzylammonium, Hydroxyethyltrimethylammonium, Chlorethyltrimethylammonium; oder Allylammonium, Diallylammonium, Cyclohexylammonium, Menthylammonium, Aminoethylammonium, Ethylendiammonium, Benzhydrylammonium, Pyrrolidinium, Morpholinium, 3-Pyridylammonium, Piperidinium oder Piperazinium, oder ein von einer Aminsäure oder deren Ester abgeleitetes Ammonium wie $NH_3$-$CH_2$-$COOCH_3$.

Entsprechend enthalten organisch substituiertes Phosphonium, organisches Sulfonium oder organisches Sulfoxonium aliphatische oder arylaliphatische Reste.

Andere Stickstoff-Kationen sind beispielsweise Hydrazonium, Hydroxylammonium, Guanidinium, Aminoguanidinium oder deren Substitutionsprodukte.

Gegenstand der Erfindung ist ferner ein Verfahren zur Herstellung der Verbindungen der Formel I und ihrer Salze oder Quaternisierungsprodukte, dadurch gekennzeichnet, daß man

a) einen Bisformylester der Formeln II a oder II b

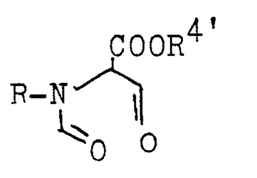

II a

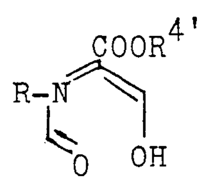

II b

wobei $R^{4'}$ = $(C_1$-$C_{12})$Alkyl bedeutet,

$(a_1)$ mit einem $(C_1$-$C_3)$Carbonsäureamid cyclisiert oder

$(a_2)$ mit Kaliumrhodanid zu einem 2-Mercaptoimidazol-Derivat umsetzt und dieses mit Salpetersäure und Natriumnitrit entschwefelt, oder

b) ein Phenylimidazolderivat der Formel III

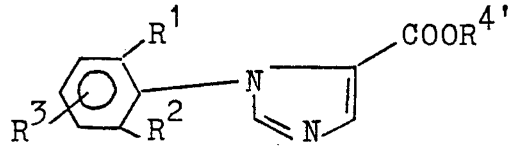

III

partiell oder vollständig im Phenylring hydriert und die unter a) oder b) erhaltenen Verbindungen derivatisiert.

Bei der Derivatisierung wird der Rest -COOR⁴ in bekannter Weise modifiziert z.B. durch Verseifung, Veresterung, Umesterung, Amidierung, Salzbildung, Reduktion oder Oximierung wie dies z.B. in den Patentanmeldungen DE-A 34 44 918 und DE-A 34 42 690 beschrieben ist, oder es erfolgt Salzbildung oder Quaternisierung am basischen Stickstoffatom des Imidazolringes.

Im Verfahren (a) wird als Carbonsäureamid vorzugsweise Formamid eingesetzt. Es wird bevorzugt in Gegenwart von Mineralsäure in molaren Mengen bei 50° -200°C, insbesondere 100° - 170°C umgesetzt.

Die Bisformylverbindungen der Formel II a bzw. II b lassen sich leicht nach bekannten Verfahren (DE-OS 32 17 094) aus 2,6-Dialkylcyclohexylaminen (DE-OS 31 23 731) oder 2,6-Dialkylcyclohexenylaminen (US-Patentschrift 43 51 667) herstellen.

Die Phenylimidazolverbindungen III sind ebenfalls literaturbekannt (DE-A 32 17 094). Als Katalysatoren für die Hydrierung lassen sich Edelmetallhydrierkatalysatoren wie z.B. Platin-, Palladium- oder Rhodiumkatalysatoren verwenden. Die Umsetzungen können in Gegenwart von Lösemitteln, wie Tetrahydrofuran, Ethanol, Methanol oder Essigsäureethylester, vorteilhaft unter Druck bei Temperaturen von 0 -200°C, insbesondere 20 -150°C durchgeführt werden.

Die Salze der Verbindungen der Formel (I) können in einfacher Weise nach üblichen Salzbildungsmethoden, z.B. durch Lösen einer Verbindung der Formel (I) in einem geeigneten organischen Lösemittel und Hinzufügen der Säure erhalten werden und in bekannter Weise, z.B. durch Abfiltrieren, isoliert und gegebenenfalls durch Waschen mit einem inerten organischen Lösemittel gereinigt werden.

Mit den erfindungsgemäßen Verbindungen sind typische wachstumregulierende Effekte erzielbar, die -verglichen den aus der DE-A 32 17 094 bekannten Vebindungen -in verschiedenen Kulturen bereits bei niederen Dosierungen eingesetzt werden können. Sie greifen regulierend in den pflanzeneigenen Stoffwechsel ein und können damit zur gezielten Beeinflussung von Pflanzninhaltsstoffen sowie zur Ernteerleichterung wie zum Auflösen von Desikkation und Wuchsstauchung eingesetzt werden. Des weiteren eignen sie sich zur generellen Steuerung und Hemmung von unerwünschtem vegetativen Wachstum, ohne dabei die Pflanzen abzutöten. Eine Hemmung des vegetativen Wachstums spielt bei vielen mono-und dikotylen Kulturen eine große Rolle, da das Lagern hierdurch verringert oder völlig verhindert werden kann. Besonders hervorzuheben ist die wachstumregulatorische Wirksamkeit der Verbindungen als Wuchshemmer in Getreide, Mais, Soja, Tabak, Baumwolle, Ackerbohne, Raps, Reis, Rasen sowie ihre Fähigkeiten, den Gehalt an erwünschten Inhaltsstoffen wie Kohlehydraten (z.B. Zuckerrohr oder Hirsekulturen) und Protein bei Nutzpflanzen zu erhöhen. Schließlich zeigen die Verbidungen eine sehr gute Verbesserung der Fruchtabzission, insbesondere bei Zitrusfrüchten.

Die erfindungsgemäßen Verbindungen der Formel I weisen außerdem eine ausgezeichnete herbizide Wirksamkeit gegen ein breites Spektrum wirtschaftlich wichtiger mono-oder dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf-oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben - schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Waschstumsstop ein, und die Unkrautpflanzen bleiben in dem zum Applikationszeitpunkt vorhandenen Wuchsstadium stehen oder sterben nach einer gewissen Zeit mehr oder weniger schnell ab, so

daß auf diese Weise eine für die Kulturpflanzen - schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono-oder dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzen.

Besonders gute herbizide Wirksamkeit wird erreicht, wenn die erfindungsgemäßen Verbindungen im Wasserreisanbau eingesetzt werden. Sie zeigen unter diesen Bedingungen eine breite Wirkung gegen eine Reihe von Reisunkräutern wie z.B. gegen Arten aus der Gruppe der Cyperaceen z.B. Eleocharis, Cyperus, Scirpus oder gegen Arten wie Sagittaria, Pistia, gegen dikotyle Unkräuter und besonders auch gegen Gräser wie Echinochloa crus galli.

Die Kulturpflanze Reis toleriert die erfindungsgemäßen Verbindungen völlig, so daß diese zur selektiven Bekämpfung von Unkräutern in Reis im Vorauflauf-und im Nachauflaufverfahren eingesetzt werden können.

Die erfindungsgemäßen Verbindungen zeichnen sich insbesondere dadurch aus, daß sie zahlreiche Samenunkräuter und auch schwer bekämpfbare Unkräuter, die aus Dauerorganen wie Knollen oder Rhizomen keimen, in sehr wirksamen und in kleinen Aufwandmengen bekämpfen und den Reis in keiner Weise schädigen.

Die erfindungsgemäßen Verbindungen der Formel I zeichnen sich ferner durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich wichtiger, phytopathogener Pilze, wie z.B. Piricularia oryzae, Pellicularia sasakii, verschiedene Rostarten, Venturia inaequalis, Cercospora-Arten und Echte Mehltaupilze im Obst-, Gemüse-, Getreide-und Zierpflanzenbau, Botrytis cinerea, Pseudocercosporella herpotrichoides, Fusarium-Arten sowie einige zu den phycomyceten zählende Pilze wie z.B. Plasmorpara viticola und Pseudoperonospora cubensis.

Die Verbindungen der Formel I eignen sich auch für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr-und Schneidölen.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Ein weiterer Gegenstand der Anmeldung sind auch Pflanzenschutzmittel, die sich durch einen wirksamen Gehalt mindestens einer Verbindung der allgemeinen Formel (I) auszeichnen.

Die erfindungsgemäßen Verbindungen lassen sich bei ihrem praktischen Einsatz gegebenenfalls auch vorteilhaft mit bekannten Wachstumsregulatoren kombinieren. Solche bekannten Wachstumregulatoren sind die Verbindungen der Formel

$R\text{-}CH_2\text{-}CH_2\text{-}N^+(CH_3)_3Cl^-$ (IV)

worin R= OH oder Cl bedeutet (common name Chlormequat für R= Cl), ferner N,N-Dimethylpiperidiniumchlorid (V, Mepiquat, Chlorid, α-Cyclopropyl-4-methoxy-α-(pyrimidin-5-yl)-benzylalkohol (VI, Ancymidol), (3aα, 4β, 4aα, 6aα, 7β, 7aα)-1-(4-chlorophenyl)-3a, 4, 4a, 6a, 7, 7a-hexahydro-4,7-methano-1H-[1,2]diazeto[3,4-f]-benzotriazol (VII, Tetcylacis), Bernsteinsäure-mono-2,2-dimethylhydrazid (VIII, Diaminoazide), 6-Hydroxy-2H-pyridazin-3-on (IX, Maleinsäurehydrazid) 2-Chlor-9-hydroxy-9H-fluoren-9-carbonsäure (X, Chlorflurenol), 5'-(Trifluormethansulfonamido)acetat-2'.4'-xylidid (XI, Mefluidid), 2-Chloroethylphosphonsäure (XII, Ethephon).

Die wachstumregulatorischen Wirkungen der Verbindungen der Formeln (IV) bis (XII) sind beschrieben in Plant Growth Regulator Handbook of the Plant Growth Regulator Working Group 2d. Ed. 1981.

Anstelle der Verbindungen der Formeln (VI) und (V) können prinzipiell auch vergleichbare Salze, die anstelle des Chloridions ein anderes übliches Anion wie Bromid, Nitrat oder 1/2 Sulfat enthalten, eingesetzt werden.

Bei der Kombination der Verbindungen der Formel (I) mit den Verbindungen der Formeln (IV) bis (XII) zeigten sich überraschenderweise auffallende synergistische Wirkungen. So lassen sich diese Kombinationen in noch geringeren Dosierungen einsetzten, als von der Wirkung der Einzelkomponenten zu erwarten war, um die gewünschten Effekte zu erzielen. Mit den Kombinationen

lassen sich auch Wildwuchserscheinungen vermindern, so daß die Kombinationen auch in der Landschaftspflege eingesetzt werden können. Desweiteren eignen sich die Kombinationen hervorragend zur generellen Steuerung und Hemmung von unerwünschten vegetativen Wachstum, wie Seitentriebbildung, ohne die Pflanzen abzutöten. Die Verbindungen der Formel (I) können auch vorteilhaft mit zwei verschiedenen Verbindungen der Formeln (IV) bis (XII) kombiniert werden.

Die Mischungsverhältnisse der Komponenten der allgemeinen Formel (I) zu den Verbindungen der Formeln (IV) bis (XII) können innerhalb weiter Grenzen, etwa zwischen 250 : 1 bis 1 : 10, - schwanken. Die Wahl des Mischungsverhältnisses ist abhängig von der Art der Mischungspartner, vom Ent wicklungsstadium der Pflanzen als auch vom Grad der gewünschten wachstumregulatorischen Wirkung. Vorzugsweise werden Mischungsverhältnisse von 10 : 1 bis 1 : 10 gewählt.

Die Kombinationen können sowohl als Mischformulierungen der Komponenten vorliegen, die dann in üblicher Weise mit Wasser verdünnt zur Anwendung gebracht werden; oder sie können als sogenannte Tankmischungen durch gemeinsame Verdünnung der getrennt formulierten Komponenten mit Wasser hergestellt werden; es besteht auch die Möglichkeit, die Komponenten nacheinander zur Anwendung zu bringen, d.h. es werden dann die Komponenten in Einzelformulierungen appliziert.

Die Verbindungen der allgemeinen Formel (I) können auch mit natürlichen oder pflanzlichen Hormonen wie Auxinen oder Cytokinen kombiniert werden.

Die erfindungsgemäßen Verbindungen der allgemeinen Formel (I), können, gegebenenfalls im Gemisch mit weiteren Wirkkomponenten, wie den Verbindungen der Formeln II bis XII, als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem (den) Wirkstoff(en) außer gegebenenfalls einem Verdünnungs-oder Inertstoff noch Netzmittel wie polyoxethylierte Fettalkohole, Alkyl-oder Alkylphenylsulfonate und/oder Dispergierhilfsmittel wie ligninsulfonsaures Natrium, 2,2'-dinaphthylmethan-6,6'-disulfonsaures Natrium, dibutylnaphtalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des (der) Wirkstoffe(s) in einem inerten organischen Lösemittel wie Butanol, Cyclohexanon, Dimethylformamid, Xylol oder auch höhersiedenden Aromaten oder aliphatischen oder cycloaliphatischen Kohlenwasserstoffen unter Zusatz von einem oder mehreren Emulgatoren hergestellt werden. Bei flüssigen Wirkstoffen kann der Lösemittelanteil auch ganz oder teilweise entfallen. Als Emulgatoren können beispielsweise verwendet werden: Alkyl-arylsulfonsaure Calciumsalze wie Cadodecylbenzolsulfonat oder nichtionische Emulgatoren wie Fettsäurepolyglykolester, Alkylarylpolyglykolether, Fettalkohlpolyglykolether, Propylenoxid-Ethylenoxid-Kondensationsprodukte, Fettalkohl-Propylenoxid-Kondensationsprodukte, Alkylpolyglykolether, Sorbitanfettsäureester, Polyoxethylensorbitanfettsäureester oder Polyoxyethylensorbitester.

Stäubemittel kann man durch Vermahlen des - (der) Wirkstoffe(s) mit feinverteilten, festen Stoffen, z.B. Talkum, natürlichen Tonen wir Kaolin, Bentonit, Pyrophillit oder Diatomeenerde erhalten. Granulate können entweder durch Verdüsen der (des) Wirkstoffe(s) auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise -gewünschtenfalls in Mischung mit Düngemitteln -granuliert werden.

In Spritzpulvern beträgt die Wirkstoffkonzentration etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 0,5 bis 20 Gew.-% an Wirkstoff(en), ver, sprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden. Daneben enthalten die genannten Wirkstoffformulierungen gegebenenfalls die jeweils üblichen Haft-, Netz-, Dispergier-, Emulgier-, Penetrations-, Lösemittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser.

Staubförmige und granulierte Zubereitungen sowie versprühbare Lösungen werden vor der Anwendung üblicherweise nicht mehr mit weiteren inerten Stoffen verdünnt.

Die Aufwandmengender Wirkstoffe der Formel I, können je nach Indikation in weiten Grenzen variieren. Bei der Anwendung als Pflanzenwachstumsregulatoren oder als Fungizide liegen sie im allgemeinen zwischen 0,02 und 1,5 kg Wirstoff pro ha. Im Falle des Einsatzes als Herbizide variieren sie bevorzugt zwischen 0,05 und 3,0 kg Wirkstoff/ha.

Formulierungsbeispiele

Beispiel 1

Ein Stäubemittel wird erhalten, indem man a) 10 GT[1)]Wirkstoff(e) und 90 GT Talkum oder einem anderen Inertstoff mischt und in einer Schlagmühle zerkleinert, oder indem man b) 60 GT Wirkstoff, 35 GT Talkum und 5 GT Haftmittel (z.B. ein Polysaccharid wie (R) Rhodopol der Rhone-Poulenc (S.A.) in der gleichen Weise homogenisiert.

Beispiel 2

Ein in Wasser leicht dispergierbares, benetzbares Pulver wird erhalten, indem man 25 GT Wirkstoff(e), 64 GT kaolinhaltigen Quarz als Inertstoff, 10 GT ligninsulfonsaures Kalium und 1 GT oleoylmethylaurinsaures Natrium als Netz-und Dispergierhilfsmittel mischt und in einer Stiftmühle mahlt. Eine Formulierung mit 5 % Wirkstoffgehalt kann wie folgt zusammengesetzt sein: 5 % Wirkstoff(e). 6 % eines sulfonierten Napthalinformaldehydkondensats (z.B. (R)Dispersogen A der Hoechst AG), 2 % eines Na-Salzes einer Alkylnapthalinsulfonsäure (z.B. (R)Leonil DB der Hoechst AG), 5 % eines Gemisches aus Polypropylenglykol und $SiO_2$(z.B. (R)Acrotin 341 der Hoechst AG), 25 % eines $SiO_2$ (z.B. (R)Sipernat der Degussa AG) und 57 % Kaolin Typ 1777.

Beispiel 3

Ein in Wasser leicht dispergierbares Dispersionskonzentrat wird erhalten indem man 20 GT Wirkstoff(e) mit 6 GT eines Alkylphenolpolyglykolethers (z.B. (R)Triton X 207 von Rohm and Haas Co.), 3 GT Isotridecanolpolyglykolether (8 Ethylenoxid-Einheiten) und 71 GT paraffinischem Mineral-81 (Siedebereich ca. 255 bis über 377 °C) mischt und in einer Reibkugelmühle auf eine Feinheit von unter 5 um vermahlt.

Beispiel 4

Ein emulgierbares Konzentrat wird erhalten aus 15 GT Wirkstoff(e), 75 GT Cyclohexanon als Lösemittel und 10 GT oxethyliertem Nonylphenol - (10 Ethylenoxid-Einheiten) als Emulgator.

[1)] GT = Gewichtsteile

Chemische Beispiele

Beispiel 1

Ethyl-1-(2,6-diethylcyclohexyl)-imidazol-5-carboxylat

39,7 g (0,13 Mol) Ethyl-2-(N-formyl-2,6-diethylcyclohexylamino)-3-hydroxy-acrylat wurden mit 240 ml Formamid und 24 ml konz. Salzsäure 6 h auf 150 °C erhitzt. Nach dem Erkalten extrahierte man zweimal mit Diisopropyläther, wusch die organische Phase zweimal mit Wasser, trocknete über Natriumsulfat, dampfte ein und chromatographierte den Rückstand über. eine Kieselgelsäule (Laufmittel Petrolether (tief) -Essigester 7 : 3). Man erhielt 20,5 g (55 % der Theorie) Ethyl-1-(2,6-diethylcyclohexyl)-imidazol-5-carboxylat, ein farbloses Öl. Die Identifizierung erfolgte NMR-spektroskopisch.

Beispiel 2

Methyl-1-(2,6-dimethylcyclohexyl)-imidazol-5-carboxylat

Zu 29,7 g (0,10 Mol) Methyl-2-(N-formyl-2,6-dimethylcyclohexylamin)-3-hydroxy-acrylate in 200 ml Tetrahydrofuran gab man eine Lösung von 19,4 g (0,20 Mol) Kaliumrhodanid in 100 ml Wasser zu. Nach dem Zutropfen von 22 g konz. Salzsäure erhitzte man 12 h auf 60 °C. Nach dem Erkalten trennte man die organische Phase ab und dampfte ein. Man nahm in 50 ml Methylenchlorid auf und tropfte diese Lösung bei 35 °C unter Stickstoffatmosphäre zu einer Lösung von 200 ml 15 % Salpetersäure, 200ml Methylenchlorid und 5 g Natriumnitrit zu. Man rührte 1 h bei 35 °C nach, kühlte auf 0 -5 °C ab und saugte das Nitrat ab. Zur Freisetzung des Imidazols wurde in Methylenchlorid suspendiert und das Nitrat mit 1-N-Natronlauge neutralisiert. Nach dem Trocknen und Eindampfen der organischen Phase erhielt man 18,3 g (66 % der Theorie) Methyl-1-(2,6-dimethylcyclohexyl)-imidazol-5-carboxylat als farbloses Öl. Die Identifizierung erfolgte NMR-spektroskopisch.

Nach den vorstehend beschriebenen Verfahren lassen sich die folgenden Beispiele herstellen. Stehen in den Tabellen 1 und 2 für X andere Reste als $-COOCH_3$, oder $COOC_2H_5$ wurden diese nach gängigen, allgemein bekannten Verfahren aus den entsprechenden Estern hergestellte.

Tabelle 1 Cyclohexylderivate

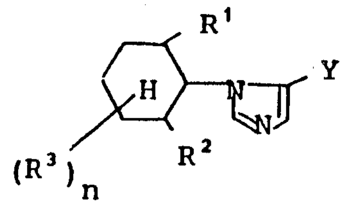

| Beispiel-Nr. | $R^1$ | $R^2$ | $(R^3)_n$ | Y | Fp(°C)/Sdp(°C) |
|---|---|---|---|---|---|
| 3 | $CH(CH_3)_2$ | $CH(CH_3)_2$ | H | $-COO^- \cdot 1/2\ Zn$ | |
| 4 | $CH_3$ | $CH_3$ | " | $-COOH$ | 185-91 |
| 5 | " | " | " | $-COOCH_2-COOCH_3$ | Öl |
| 6 | " | " | " | 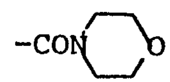 | 93-8 |
| 7 | " | " | " | $-C(=S)-NH_2$ | |
| 8 | " | " | $4-CH_3$ | $-CHO$ | Öl |
| 9 | " | " | " | $-CH(OC_2H_5)_2$ | Öl |
| 10 | " | " | " | $-CH_2-OH$ | |
| 11 | " | " | $4-OCH_3$ | $-COO-CH_2-S-CH_3$ | |
| 12 | $CH_3$ | $C_2H_5$ | H | $-COOH$ | 213-6 |
| 13 | " | " | " | 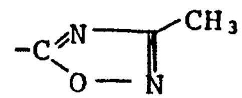 | |
| 14 | " | " | " | 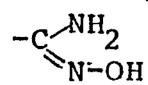 | |
| 15 | " | " | " | $-C\equiv N$ | Öl |

Fortsetzung Tabelle 1

| Beispiel-Nr. | $R^1$ | $R^2$ | $(R^3)_n$ | Y | Fp(°C) / /Sdp(°C) |
|---|---|---|---|---|---|
| 16 | $CH_3$ | $C_2H_5$ | H | 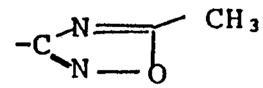 | Öl |
| 17 | $C_2H_5$ | " | " | $-COOH$ | 170-4 |
| 18 | " | " | " | $-COOH \cdot NH_3$ | 198 (Zers.) |
| 19 | " | " | " | $-COONa$ | >240 (Zers.) |
| 20 | " | " | " | $-COOK$ | >220 (Zers.) |
| 21 | " | " | " | $-COOH \cdot H_2N-(CH_2)_3-O-CH_3$ | 107-11 |
| 22 | " | " | " | $-COOH \cdot N(-CH_2-CH_2-OH)_3$ | Harz |
| 23 | " | " | " | $-COOH \cdot H_2N-CH_2-COOCH_3$ | Harz |
| 24 | " | " | " | $-COOH \cdot$ 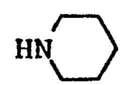 | Harz |
| 25 | " | " | " | $-COOH \cdot H_2N-NH-C(=O)-NH_2$ | 152-8 |
| 26 | " | " | " | $-COOH$ (Hydrochlorid) | >205 (Zers.) |
| 27 | " | " | " | $-COOH$ (Nitrat) | >188 (Zers.) |
| 28 | " | " | " | $-COOH$ (2-Chlorethyl-phosphonat) | 102-5 |
| 29 | " | " | " | $-COOCH_3$ | Öl |
| 30 | " | " | " | $-COOCH(CH_3)_2$ | Öl |
| 31 | " | " | " | $-COO-N=C(CH_3)_2$ | Öl |
| 32 | " | " | " | $-C(=S)-OC_2H_5$ | Öl |

----------

Fortsetzung Tabelle 1

| Beispiel-Nr. | $R^1$ | $R^2$ | $(R^3)_n$ | Y | Fp(°C)/ Sdp(°C) |
|---|---|---|---|---|---|
| 33 | $C_2H_5$ | $C_2H_5$ | 4-Br | $-COOH$ | |
| 34 | " | " | " | $-COOH$ · 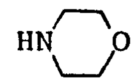 | |
| 35 | " | $-CH(CH_3)_2$ | H | $-COOH$ | 142-9 |
| 36 | " | " | " | $-CN$ | |
| 37 | " | " | " | $-COCH_3$ | |
| 38 | " | " | " | $-CH=NOH$ | |
| 39 | " | " | " | $-C(=O)-S-C_2H_5$ | |
| 40 | " | " | " | -COOPhenyl | |
| 41 | " | " | " | $-CONH_2$ | Öl |
| 42 | " | " | " | $-C(=O)-NH-OH$ | |
| 43 | " | " | " | $-C(=O)NH-NH_2$ | |
| 44 | " | " | " | $-C(=O)NH-N(CH_3)_2$ | |
| 45 | " | " | " | 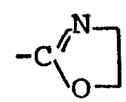 | |
| 46 | " | " | " | 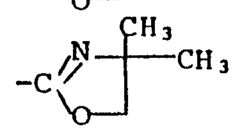 | |
| 47 | " | " | " | $-COO-CH_2-CN$ | |
| 48 | " | " | " | $-COO-CH_2-COOCH_3$ | |

...

Fortsetzung Tabelle 1

| Beisp.-Nr. | $R^1$ | $R^2$ | $(R^3)n$ | Y | Fp[°C]/Sdp(°C) |
|---|---|---|---|---|---|
| 49 | $CH_3$ | $C_2H_5$ | H | $-COOCH_3$ | Öl |
| 50 | " | " | " | $-COOC_2H_5$ | Öl |
| 51 | " | " | " | $-COOH \cdot N(-CH_2-CH_2-OH)_3$ | Harz |
| 52 | " | " | " | $-COOH \cdot$ 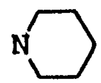 | Harz |
| 53 | $C_2H_5$ | " | " | $-CONH_2$ | Öl |
| 54 | " | " | " | $-C{\equiv}N$ | Öl |
| 55 | " | " | " | $-C(=NOH)NH_2$ | 127-133 |
| 56 | " | " | " | $-COO-CH_2-CH{=}CH_2$ | Öl |
| 57 | " | " | " | " (Hydrochlorid) | 125-130 |
| 58 | " | " | " | $-COO-CH_2-CCl_3$ | Öl |
| 59 | " | " | " | $-CONH-CH_2-CH(OCH_3)_2$ | 42-45 |
| 60 | " | " | " | $-CONH-CH_2-CHO$ | Öl |
| 61 | " | " | " | $-C(=O)-NHOH$ | Harz |
| 62 | " | " | " | $-C(=S)-NH_2$ | 112-115 |
| 63 | " | " | " | $-COO-N=C(CH_3)_2$ (Hydrochlorid) | Harz |
| 64 | " | " | " | $-CONH-CH_3$ | Öl |

Fortsetzung zu Tabelle 1

| Beispiel-Nr. | $R^1$ | $R^2$ | $(R^3)_n$ | Y | Fp / Sdp (°C) |
|---|---|---|---|---|---|
| 65 | $C_2H_5$ | $C_2H_5$ | H | $-CONH-CH(CH_3)_2$ | Öl |
| 66 | " | " | " | -CON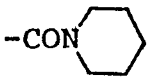 | Öl |
| 67 | " | " | " | $-CH_2OH$ | Öl |
| 68 | " | " | " | -CHO | Öl |
| 69 | " | " | " | -CHNOH | Öl |
| 70 | " | " | " | $-C(=O)-NHOCH_3$ | Öl |
| 71 | " | " | " | $-COO-CH(CH_3)-n-C_6H_{13}$ | Öl |
| 72 | " | " | " | -COO-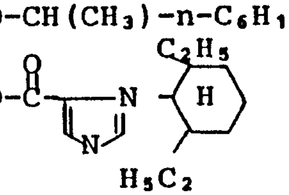 | 98-101 |
| 73 | " | " | " | 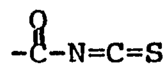 | Öl |
| 74 | " | " | " | $-CON(C_2H_5)_2$ | |
| 75 | " | " | " | $-CO-N(CH_3)$Phenyl | |
| 76 | " | " | " | $-CO-N$(Cyclohexyl)$-i-C_3H_7$ | |
| 77 | " | " | " | $-CON(CH_2-CH=CH_2)_2$ | |
| 78 | " | " | " | $-CONH(C_9H_{19})$ | |
| 79 | " | " | " | $-CONH-CH_2$-cyclohexyl | |
| 80 | " | " | " | CONH-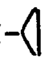 | |
| 81 | " | " | " | -CO-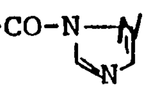 | |

**Tabelle 2** Cyclohexenylderivate

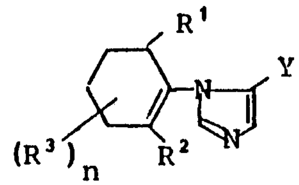

| Beispiel-Nr. | $R^1$ | $R^2$ | $(R^3)_n$ | Y | Fp(°C) / Sdp(°C) |
|---|---|---|---|---|---|
| 82 | $CH_3$ | $CH_3$ | H | COOH | |
| 83 | " | " | " | $COOH \cdot 1/2(H_2N-CH_2-CH_2-NH_2)$ | |
| 84 | " | " | " | $COOCH_3$ | |
| 85 | " | " | " | $COOCH_2-CH=CH_2$ | |
| 86 | $C_2H_5$ | $C_2H_5$ | " | COOH | |
| 87 | " | " | " | -COO-Cyclohexyl | |
| 88 | " | " | " | $-CONH-CH_2-CH_2-OH$ | |
| 89 | " | " | " | 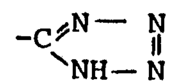 | |
| 90 | $CH(CH_3)_2$ | $CH(CH_3)_2$ | " | COOH | |
| 91 | " | " | " | COOLi | |
| 92 | " | " | " | COO · 1/2Mg | |
| 93 | " | " | " | 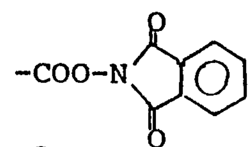 | |
| 94 | " | " | " | 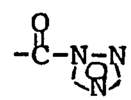 | ... |
| 95 | " | " | " | $-COOCH_2-CF_3$ | |

Biologische Beispiele

A. Wachstumregulierung

I. Wuchshemmung an Getreide

In Schalenversuchen im Gewächshaus wurden junge Getreidepflanzen (Weizen, Gerste, Roggen) im 3-Blattstadium mit erfindungsgemäßen Verbindungen in verschiedenen Wirkstoffkonzentrationen (kg/ha) tropfnaß gespritzt.

Nachdem die unbehandelten Kontrollpflanzen eine Wuchshöhe von etwa 55 cm erreicht hatte, wurde bei allen Pflanzen der Zuwachs gemessen und die Wuchshemmung in % des Zuwachses der Kontrollpflanzen berechnet. Es wurde außerdem die phytotoxische Wirkung der Verbindung beobachtet. Die Wuchshemmung wurde als prozentualer Wert ermittelt, wobei 100 % den Stillstand des Wachstums und 0 % ein Wachstum entsprechend dem der unbehandelten Kontrollpflanzen bedeuten. Es zeigte sich, daß die Verbindungen sehr gute wachstumsregulierende Eigenschaften besitzen. Die Ergebnisse sind in Tabelle III zusammengefaßt.

II. Wuchshemmung in Wasserreis

Reispflanzen wurden angezogen und im Stadium der maximalen Bestockung mit erfindungsgemäßen Verbindungen behandelt. Die Substanzen wurden sowohl durch Spritzung appliziert als auch in das Wasser gegeben.

3 Wochen nach Behandlung wurde bei allen Pflanzen der Zuwachs gemessen und die Wuchshemmung in % des Zuwachses der Kontollpflanzen berechnet. Es wurde außerdem auf eine mögliche phytotoxische Wirkung der Verbindungen geachtet.

Die Wuchshemmung wurde als prozentualer Wert ermittelt, wobei 100 % den Stillstand des Wachstums und 0 % ein Wachstum entsprechend dem der unbehandelten Kontrollpflanzen bedeuten. Es zeigte sich, daß die Verbindungen sehr gute wachstumsregulierenden Eigenschaften besitzen. Die Ergebnisse sind in Tabelle III zusammengefaßt.

III. Wuchshemmung an Sojabohnen

Ca. 10 cm große Sojabohnen wurden mit den Wirkstoffzubereitungen tropfnaß bespritzt. Nach 3 Wochen wurde bonitiert.

Die Wuchshemmung wurde als prozentualer Wert ermittelt, wobei 100 % den Stillstand des Wachstums und 0 % ein Wachstum entsprechend dem der unbehandelten Kontrollpflanzen bedeuten. Es zeigte sich, daß die Verbindungen sehr gute wachstumsregulierenden Eigenschaften besitzen. Die Ergebnisse sind in der nachfolgenden Tabelle III zusammengefaßt.

Tabelle III

| Verbindung nach Beispiel Nr. | Anwendungs-konz. (kg/ha) | Wuchshemmung in % bei | | | | |
|---|---|---|---|---|---|---|
| | | Reis | Weizen | Gerste | Roggen | Soja |
| 1 | 1,0<br>0,75 | 14<br>9 | 12<br>9 | 10<br>8 | 11<br>7 | 9<br>6 |
| 2 | 1,0<br>0,75 | 12<br>9 | 12<br>9 | 11<br>8 | 10<br>7 | 9<br>6 |
| 4 | 1,0<br>0,75 | 18<br>11 | 11<br>9 | 11<br>8 | 10<br>9 | 11<br>9 |
| 8 | 1,0<br>0,75 | 11<br>7 | 12<br>7 | 10<br>7 | 11<br>8 | 9<br>8 |
| 12 | 1,0<br>0,75 | 19<br>17 | 12<br>10 | 11<br>9 | 12<br>9 | 10<br>8 |
| 13 | 1,0<br>0,75 | 12<br>8 | 10<br>8 | 11<br>9 | 11<br>9 | 12<br>8 |
| 16 | 1,0<br>0,75 | 13<br>7 | 11<br>8 | 11<br>8 | 12<br>8 | 11<br>9 |
| 17 | 1,0<br>0,75 | 19<br>17 | 12<br>11 | 11<br>8 | 11<br>9 | 11<br>9 |
| 18 | 1,0<br>0,75 | 18<br>17 | 12<br>11 | 12<br>8 | 12<br>9 | 11<br>7 |
| 19 | 1,0<br>0,75 | 22<br>17 | 13<br>10 | 12<br>10 | 9<br>7 | 12<br>7 |
| 21 | 1,0<br>0,75 | 19<br>17 | 12<br>11 | 10<br>10 | 12<br>9 | 11<br>7 |
| 22 | 1,0<br>0,75 | 23<br>20 | 14<br>7 | 13<br>7 | 12<br>8 | 9<br>7 |
| 24 | 1,0<br>0,75 | 18<br>16 | 12<br>9 | 12<br>10 | 13<br>11 | 12<br>8 |
| 26 | 1,0<br>0,75 | 20<br>18 | 14<br>9 | 12<br>9 | 13<br>9 | 9<br>9 |
| 28 | 1,0<br>0,75 | 17<br>16 | 13<br>10 | 14<br>9 | 20<br>9 | 12<br>7 |
| 29 | 1,0<br>0,75 | 13<br>10 | 12<br>7 | 11<br>8 | 13<br>9 | 12<br>11 |
| 30 | 1,0<br>0,75 | 13<br>9 | 12<br>7 | 12<br>8 | 11<br>9 | 13<br>7 |
| 31 | 1,0<br>0,75 | 23<br>20 | 14<br>13 | 14<br>9 | 13<br>10 | 13<br>10 |

Fortsetzung Tabelle III

| Verbindung nach Beispiel Nr. | Anwendungs-konz. (kg/ha) | Wuchshemmung in % bei | | | | |
|---|---|---|---|---|---|---|
| | | Reis | Weizen | Gerste | Roggen | Soja |
| 33 | 1,0 | 22 | 16 | 16 | 14 | 12 |
| | 0,75 | 20 | 12 | 10 | 10 | 10 |
| 41 | 1,0 | 14 | 12 | 12 | 13 | 13 |
| | 0,75 | 9 | 11 | 10 | 9 | 8 |
| 42 | 1,0 | 15 | 13 | 12 | 12 | 13 |
| | 0,75 | 11 | 10 | 9 | 9 | 7 |

Für keine der getesteten Verbindungenwurden phytotoxische Effekte beobachtet.

B. Herbizide Wirkung

Die Schädigung der Unkrautpflanzen bzw. die Kulturpflanzenverträglichkeit wurde gemäß einem Schlüssel bonitiert, in dem die Wirksamkeit durch Wertzahlen von 0 -5 ausgedrückt ist. Dabei bedeutet:

0 = ohne Wirkung bzw. Schaden

1 = 0 -20 % Wirkung bzw. Schaden

2 = 20 -40 % Wirkung bzw. Schaden

3 = 40 -60 % Wirkung bzw. Schaden

4 = 60 -80 % Wirkung bzw. Schaden

5 = 80 -100 % Wirkung bzw. Schaden

IV. Unkrautwirkung im Vorauflauf

Samen bzw. Rhizomstücke von mono-und dikotylen Unkrautpflanzen wurden in Plastiktöpfen - (Durchmesser 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wässrige Suspensionen bzw. Emulsionen mit einer Wasseraufwandmenge von umgerechnet 600 -800 1/ha in unterschiedlichen Dosierungen auf die Oberfläche der Abdeckerde appliziert.

Nach der Behandlung wurden die Töpfe im Gewächshaus aufgestellt und unter guten Waschstumsbedinungen für die Unkräuter gehalten (Temperatur: 23 plus/minus 2°C; relative Luftfeuchte: 60 -80 %).

Die optische Bonitur der Pflanzen-bzw. der Auflaufschäden erfolgte nach dem Auflaufen der Versuchspflanzen nach einer Versuchszeit von 3 -4 Wochen im Vergleich zu unbehandelten Kontrollen.

Wie die Boniturwerte in Tabelle 1 zeigen, weisen die erfindungsgemäßen Verbindungen eine gute herbizide Vorauflaufwirksamkeit gegen ein breites Spektrum von Ungräsern und Unkräutern auf.

Tabelle IV

Herbizide Wirkung der erfindungsgemäßen Verbindungen im Vorauflauf

| Beispiel Nr. | Dosis kg a.i./ha | herbizide Wirkung CYI | LOM | ECG | SIA | STM | CRS |
|---|---|---|---|---|---|---|---|
| 29 | 2,4 | 5 | 5 | 5 | 4 | 5 | 5 |
| 1 | 2,4 | 5 | 2 | 5 | 4 | 5 | 4 |
| 30 | 2,4 | 5 | 2 | 3 | 3 | 3 | 3 |
| 53 | 2,4 | 5 | 5 | 5 | 5 | 5 | 5 |
| 54 | 2,4 | - | 4 | 5 | 2 | 4 | 5 |
| 56 | 2,4 | 5 | 1 | 5 | 5 | 5 | 4 |
| 31 | 2,4 | 4 | 3 | 5 | 3 | 4 | 2 |
| 63 | 2,4 | - | 3 | 4 | 2 | 5 | 2 |
| 64 | 2,4 | 5 | 5 | 5 | 5 | 5 | 5 |
| 12 | 2,4 | 3 | 2 | 4 | 4 | 3 | 2 |
| 50 | 2,4 | 5 | 1 | 5 | 4 | 5 | 3 |

**Abkürzungen:**

CYI = Cyperus iria
LOM = Lolium multiflorum
ECG = Echinochloa crus galli
SIA = Sinapis alba
STM = Stellaria media
CRS = Chrysanthemum segetum

V. Herbizidwirkung in Reis

Samen und Knollen bzw. Jungpflanzen verschiedener Reisunkräuter wie z.B. gegen Arten von Cyperus, Echinochloa, Eleocharis und Salvinia werden in geschlossenen Töpfen von 13 cm Durchmesser ausgepflanzt und mit Wasser bis zur Höhe von 1 cm über dem Boden angestaut. Ebenso wird mit Reispflanzen verfahren.

Im Vorauflaufverfahren werden die Verbindungen ins Bewässerungswasser gegossen (in Form von wässrigen Suspensionen bzw. Emulsionen oder in Form von Granulaten) bzw. ins Wasser gestreut.

Jeweils 3 Wochen später wird die herbizide Wirkung und die Schadwirkung gegenüber Reis bonitiert.

Die Ergebnisse zeigten, daß sich die erfindungsgemäßen Verbindungen zur selektiven Unkrautbekämpfung in Reis eignen. Gegenüber bisherigen Reisherbiziden zeichnen sich die erfindungsmgemäßen Verbindungen dadurch aus, daß sie zahlreiche Samenunkräuter und auch schwer bekämpfbare Unkräuter, die aus Dauerorganen wie Knollen (Cyperus!) oder Rhizomen keimen, in sehr wirksamen und in kleinen Aufwandmengen bekämpfen und den Reis in keiner Weise schädigen.

Tabelle V

| Verbindung | Dosis kg AS/ha | Reis gesät | Reis gepflanzt | Schädigung (%) ECG | CYD | EOA | CYS | SAN |
|---|---|---|---|---|---|---|---|---|
| 1 | 2 | 0 | 0 | 100 | 100 | 100 | 100 | 100 |
| | 1 | 0 | 0 | 100 | 100 | 95 | 98 | 100 |
| | 0.5 | 0 | 0 | 99 | 100 | 92 | 90 | 95 |

Die Verbindungen ach Beisp. Nr. 29, 53, 54, 56 und 50 wirken in ähnlicher Weise.

C. Fungizide Wirksamkeit

Beispiel VI

Weizenpflanzen wurden im 3-Blattstadium mit Konidien des Weizenmehltaus (Erysiphe graminis) stark inokuliert und in einem Gewächshaus bei 20°C und einer relativen Luftfeuchte von 90 -95 % aufgestellt. 3 Tage nach Inokulation wurden die Pflanzen mit den in Tabelle I aufgeführten Verbindungen und Wirkstoffkonzentrationen behandelt. Nach einer Inkubationszeit von 10 Tagen wurden die Pflanzen auf Befall mit Weizenmehltau untersucht. Der Befallsgrad wird ausgedrückt in % befallener Blattfläche, bezogen auf unbehandelte, infizierte Kontrollpflanzen (=100 % Befall). Das Ergebnis ist in der Tabelle I zusammengefaßt.

Tabelle VI

| Verbindung gemäß Beispiel Nr. | mit Weizenmehltau befallene Blattfläche in % bei mg Wirkstoff/Liter Spritzbrühe | | |
|---|---|---|---|
| | 500 | 250 | 125 |
| 67 | 0 | 0 | 0 |
| 66 | 0 | 0 | 0-3 |
| 1 | 0 | 0 | 0 |
| 31 | 0 | 0 | 0 |
| 56 | 0 | 0 | 0 |
| 63 | 0 | 0 | 0 |
| 57 | 0 | 0 | 0 |
| 54 | 0 | 0 | 0 |
| 58 | 0 | 0 | 0 |
| 50 | 0 | 0 | 0 |
| 17 | 0 | 0 | 0 |
| unbehandelte, infizierte Pflanzen | | 100 | |

Beispiel VII

Gerstenpflanzen wurden im 3-Blattstadium mit Konidien des Gerstenmehltaus (Erysiphe graminis sp. hordei) stark inokuliert und in einem Gewächshaus bei 20°C und einer relativen Luftfeuchte von 90 -95 % aufgestellt. 3 Tage nach Inokulation wurden die Pflanzen mit den in Tabelle II aufgeführten Verbindungen und Wirkstoffkonzentrationen behandelt. Nach einer Inkubationszeit von 10 Tagen wurden die Pflanzen auf Befall mit Gerstenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle II zusammengefaßt.

Tabelle VII

| Verbindung gemäß Beispiel Nr. | mit Gerstenmehltau befallene Blattfläche in % bei mg Wirkstoff/Liter Spritzbrühe | | |
|---|---|---|---|
| | 500 | 250 | 125 |
| 67 | 0 | 0 | 0 |
| 1 | 0 | 0 | 0 |
| 31 | 0 | 0 | 0 |
| 56 | 0 | 0 | 0 |
| 50 | 0 | 0 | 0-3 |
| unbehandelte, infizierte Pflanzen | | | 100 |

Beispiel VIII

Gurkenpflanzen (Sorte Delikateß wurden im 2-Blattstadium mit einer Konidiensuspension von Gurkenmehltau (Erysiphe cichoracearum) stark inokuliert. Nach einer Abtrockungszeit der Sporensuspension von 30 Minuten wurden die Pflanzen in einem Gewächshaus bei 22°C und 90 % relativer Luftfeuchte aufgestellt.

3 Tage nach Infektion wurden die Pflanzen mit den in Tabelle III genannten Verbindungen und Wirkstoffkonzentrationen behandelt. Nach 10 Tagen erfolgt die Bonitur. Der Befallsgrad wird ausgedrückt in % befallener Blattfläche, bezogen auf unbehandelte, infizierte Kontrollpflanzen (= 100 % Befall). Das Ergebnis ist in Tabelle III zusammengefaßt.

Tabelle VIII

| Verbindung gemäß Beispiel Nr. | mit Gurkenmehltau befallene Blattfläche in % bei mg Wirkstoff/Liter Spritzbrühe | | |
|---|---|---|---|
| | 500 | 250 | 125 |
| 67 | 0 | 0 | 0 |
| 1 | 0 | 0 | 0 |
| 31 | 0 | 0 | 0 |
| 56 | 0 | 0 | 0 |
| unbehandelte, infizierte Pflanzen | | 100 | |

Beispiel IX

Weizenpflanzen wurden mit den in Tabelle IV beanspruchten Verbindungen und Wirkstoffkonzentrationen behandelt. Nach dem Antrocknen des Wirkstoffbelages wurden die Pflanzen mit Sporen des Weizenbraunrostes (Puccinia triticina) inolkuliert und tropfnaß in eine Klimakammer mit 20°C und 100 % rel. Luftfeuchte gestellt. 24 Stunden später kamen die Pflanzen in ein Gewächshaus zurück und wurden hier 14 Tage nach Inokulation auf Befall mit Weizenbraunrost untersucht. Der Befallsgrad wurde ausgedrückt in % befallener Blattfläche, bezogen auf unbehandelte, infizierte Kontrollpflanzen (= 100 % Befall). Tabelle IV zeigt die gute Wirkung der untersuchten Verbindungen.

Tabelle IX

| Verbindung gemäß Beispiel Nr. | % mit Braunrost befallene Blattfläche bei mg Wirkstoff/Liter Spritzbrühe | | |
|---|---|---|---|
| | 500 | 250 | 125 |
| 67 | 0 | 0 | 0 |
| 1 | 0 | 0 | 0 |
| 31 | 0 | 0 | 0 |
| 56 | 0 | 0 | 0 |
| 57 | 0 | 0 | 0 |
| 54 | 0 | 0 | 0 |
| 65 | 0 | 0 | 0 |
| 68 | 0 | 0 | 0 |
| 50 | 0 | 0 | 0 |
| 17 | 0 | 0 | 0 |
| unbehandelte, infizierte Pflanzen | | | 100 |

Beispiel X

Biomalzagar wurde mit den in Tabellen X und XI beanspruchten Wirkstoffverbindungen und Konzentrationen versetzt. Nach dem Erstarren des Agars erfolgte die Beimpfung mit verschiedenen Botrytis cinerea-Kulturen, die Benzimidazol-und Dicarboximid-sensible bzw. -resistent sind. Jeweils 20 µl einer Sporensuspension wurden auf das Zentrum der Agarplatte ausgebracht. Die Versuche wurden 6 Tage nach Beimpfung ausgewertet. Der Wirkungsgrad der Wirkstoffe wird ausgedrückt in % im Vergleich zur Kontrolle (Agarmedium ohne Wirkstoff)

Tabelle X Botrytis cinerea, BCM- und Iprodion-sensibler Stamm

| Verbindungen gemäß Beispiel Nr. | Wirkungsgrad in % bei ppm Wirkstoff | |
|---|---|---|
| | 2000 | 500 |
| 67 | 100 | 100 |
| 58 | 100 | 100 |
| 66 | 100 | 100 |
| 68 | 100 | 100 |
| 31 | 100 | 100 |
| 56 | 100 | 100 |
| 50 | 100 | 100 |
| Kontrolle | 0 | |

<u>Tabelle XI</u> Botrytis cinerea, BCM- und Iprodion-resistener Stamm

| Verbindungen gemäß Beispiel Nr. | Wirkungsgrad in % bei ppm Wirkstoff 2000 | 500 |
|---|---|---|
| 67 | 100 | 100 |
| 1 | 100 | 100 |
| 58 | 100 | 100 |
| 66 | 100 | 100 |
| 68 | 100 | 100 |
| 31 | 100 | 100 |
| 56 | 100 | 100 |
| 50 | 100 | 100 |
| Kontrolle | 0 | |

**Ansprüche**

1. Verbindungen der Formel I

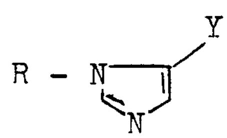

I

worin

R = einen Rest der Formeln

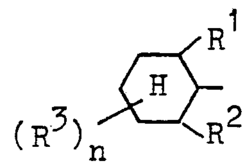

oder

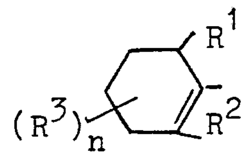

,

n= 0 bis 6,

Y=

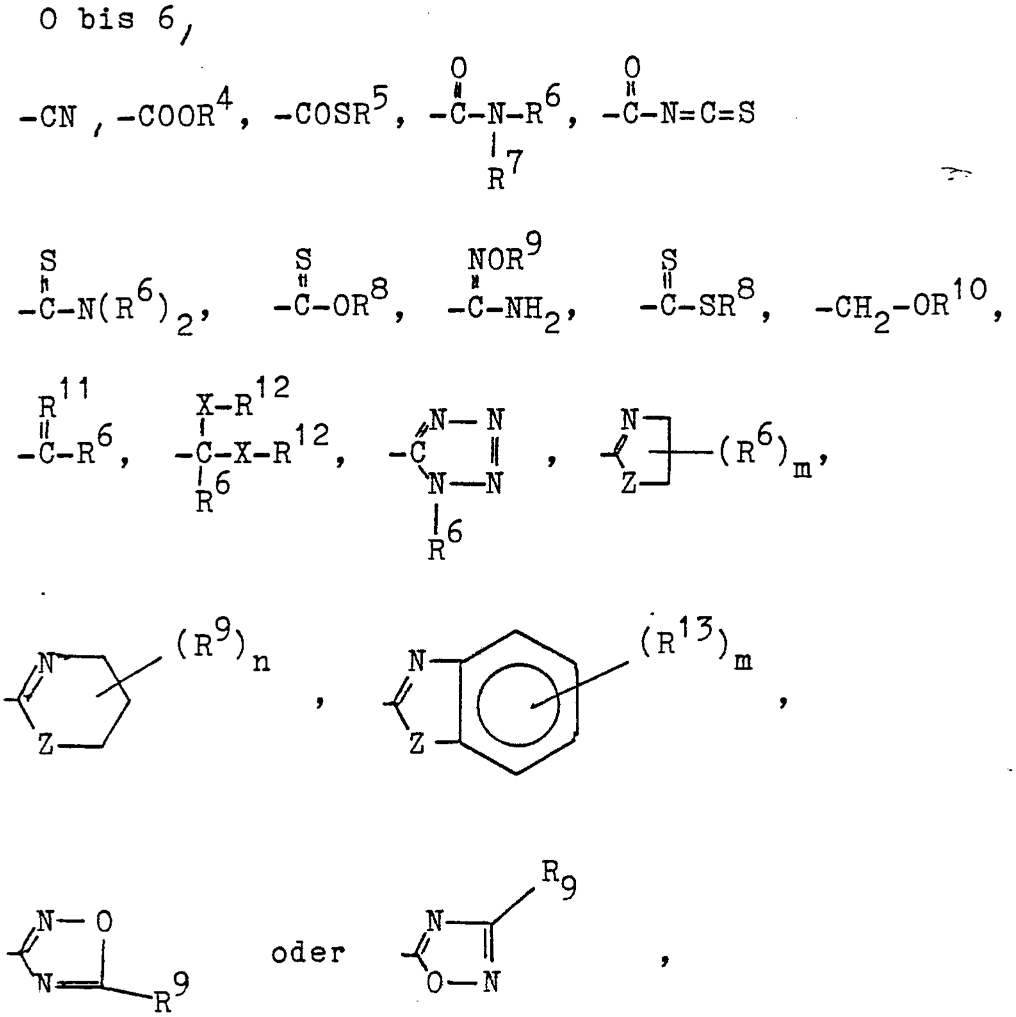

oder

m = 1 bis 4,

Z = O, S oder N-$R^6$.

X = O oder S,

$R^1$, $R^2$ unabhängig voneinander ($C_1$-$C_4$)Alkyl oder Wasserstoff,

$R^3$ jeweils unabhängig voneinander ($C_1$-$C_4$)Alkyl, -($C_1$-$C_4$)Alkoxy, ($C_1$-$C_4$)Hydroxyalkyl,

($C_1$-$C_4$)-Alkoxy($C_1$-$C_4$)alkyl, oder Halogen,

$R^4$ Wasserstoff, ($C_1$-$C_{12}$)Alkyl, ($C_1$-$C_{12}$)-Alkyl, das ein- bis dreifach substituiert ist Hydroxy, Halogen, Cyano, ($C_1$-$C_6$)Alkoxy, ($C_1$-$C_4$)Alkoxy($C_1$-$C_4$)alkoxy, -($C_1$-$C_4$)Alkylthio, ($C_1$-$C_4$)Alkylsulfinyl, ($C_1$-$C_4$)-Alkylsulfonyl, Mono-oder Di-($C_1$-$C_4$-alkyl)amino, Cyano, Aminocarbonyl, ($C_1$-$C_4$)Alkanoyl, ($C_1$-$C_4$-Alkoxy)carbonyl, Cyclo($C_3$-$C_7$)alkyl, Tri($C_1$-$C_4$)alkyl)-silyl, Benzyloxy, Benzyloxyethoxy, Phenyl oder Phenyl, das durch Halogen oder ($C_1$-$C_4$)Alkyl ein- oder mehrfach substituiert ist, oder Phenoxy, Phenylthio, die beide durch Halogen oder ($C_1$-$C_4$)-Alkyl ein-oder mehrfach substituiert sein können, ferner durch Oxiranyl, Tetrahydrofuryl, Triazolyl, Pyridinyl, Imidazolyl, Carboxy, Carboxylat mit einem für die Landwirtschaft einsetzbaren Kation oder den Rest -O-N = $C(CH_3)_2$;

($C_3$-$C_6$)Alkenyl, halogeniertes ($C_3$-$C_6$)Alkenyl, unsubstituiertes oder durch Halogen oder ($C_1$-$C_4$)Alkyl substituiertes Cyclo($C_3$-$C_7$)alkyl, unsubstituiertes

oder durch Halogen oder ($C_1$-$C_4$)Alkyl substituiertes Cyclo($C_5$-$C_7$)alkenyl, ($C_3$-$C_6$)Alkinyl, 1,2-Epoxy-prop-3-yl, Phenyl oder Phenyl, das ein oder zweifach durch Halogen, Nitro, Cyano, ($C_1$-$C_4$)Alkyl, ($C_1$-$C_4$-Alkoxy)carbonyl oder ($C_1$-$C_4$)Alkoxy substituiert ist.

($C_1$-$C_4$-Alkoxy)carbonyl, Phenylcarbonyl, wobei der Phenylring durch Halogen, Nitro, Cyano, ($C_1$-$C_4$)-Alkyl substituiert sein kann,

einen Rest der Formeln 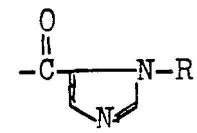,

$-N=CR^{14}R^{15}$, $-NR^{6}R^{16}$,

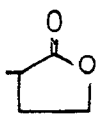, 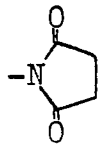, 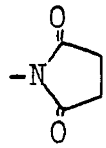 oder 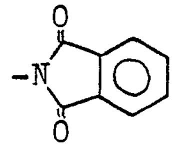;

$R^5$ ($C_1$-$C_{12}$)-Alkyl oder ($C_1$-$C_{12}$)Alkyl, das bis zu zweifach durch ($C_1$-$C_4$)Alkoxyethoxy, Cyclo($C_3$-$C_6$)alkyl, Benzyloxy, Phenyl, Phenoxy, ($C_1$-$C_4$)Alkylthio, ($C_1$-$C_4$-Alkoxy)carbonyl, Carboxy oder Carboxylat mit einem für die Landwirtschaft einsetzbares Kation, substituiert ist,

$R^6$ jeweils unabhängig voneinander Wasserstoff, ($C_1$-$C_6$)Alkyl, Phenyl oder ($C_3$-$C_6$__-Alkenyl,

$R^7$ Wasserstoff, ($C_1$-$C_{12}$)Alkyl oder ($C_1$-$C_{12}$)Alkyl, das bis zu zweifach, durch ($C_1$-$C_{12}$)Alkyl oder ($C_1$-$C_4$)-Alkoxyethoxy, Hydroxy, Halogen, Cyclo($C_3$-$C_6$)alkyl, Benzyloxy, Cyano, Aminocarbonyl, Carboxy, ($C_1$-$C_4$-Alkoxy)-carbonyl, Formyl, Phenyl oder Phenoxy substituiert ist, Phenyl oder Phenyl, das bis zu zweifach durch Halogen, Nitro, Cyano ($C_1$-$C_4$)Alkyl oder ($C_1$-$C_4$)Alkoxy substituiert ist; ($C_3$-$C_6$)-Alkenyl, -($C_3$-$C_6$))Cycloalkyl, einen Rest der Formeln $-NR^6R^{17}$, $-OR^9$, $-NH-CO-NH_2$, $-NH-CS-NH_2$ oder $-SO_2R^{18}$ oder

$R^6$ und $R^7$ bilden gemeinsam mit dem Stickstoffatom an das sie gebunden sind, einen gesättigten oder ungesättigten gegebenenfalls benzokondensierten drei-bis sieben gliedrigen Ring, der bis zu drei Heteroatome aus der Gruppe O, N und S enthält und der unsubstituiert oder durch ($C_1$-$C_4$)-Alkyl oder Halogen substituiert ist und eine Carbonylgruppe enthalten kann,

$R^8$ H, ($C_1$-$C_6$)Alkyl oder Phenyl, oder im Falle R = -CS-$OR^9$ ein für die Landwirtschaft einsetzbares Kation,

$R^9$ jeweils unabhängig voneinander H, ($C_1$-$C_4$)Alkyl oder Benzyl;

$R^{10}$ jeweils unabhängig voneinander H, unsubstituiertes oder durch Phenyl, Halogenphenyl, Nitrophenyl, Cyanophenyl, ($C_1$-$C_4$))Alkylphenyl oder ($C_1$-$C_4$)Alkoxyphenyl, Hydroxy, Cyano, ($C_1$-$C_4$-Alkoxy)-carbonyl, ($C_1$-$C_4$)Alkylthio, ($C_1$-$C_4$)Alkoxy, Cyclo($C_5$-$C_7$)alkyl oder Benzyloxy substituiertes ($C_1$-$C_{12}$)alkyl;

Cyclo($C_5$-$C_8$)alkyl, ($C_3$-$C_6$)Alkenyl, Halogen($C_3$-$C_6$)-alkenyl, ($C_3$-$C_6$)Alkinyl, Cyclo($C_5$-$C_6$)alkenyl, ($C_1$-$C_6$-Alkyl)carbonyl, Halogen($C_1$-$C_6$-alkyl)carbonyl, [($C_1$-$C_6$-Alkyl)amino]carbonyl, Benzoyl, Halogenbenzoyl oder Methylbenzoyl;

$R^{11}$ Sauerstoff (einschließlich der Bisufitaddukte) oder der Rest $=N-O-R^{10}$,

$R^{12}$ jeweils unabhängig voneinander unsubstituiertes oder durch Phenyl, Cyclo($C_5$-$C_7$)alkyl, ($C_1$-$C_4$)-Alkoxy, ($C_1$-$C_4$)Alkylthio oder Halogen substituiertes ($C_1$-$C_6$)Alkyl;

oder beide Rest $R^{12}$ gemeinsam mit X und dem Kohlenstoffatom an das sie gebunden sind, einen unsubstituierten oder durch ($C_1$-$C_4$)Alkyl, Hydroxy-($C_1$-$C_4$)alkyl, Halogen($C_1$-$C_4$)alkyl oder Phenyl substituierten 5-oder 6-gliedrigen gesättigten heterocyclischen Ring,

$R^{13}$ jeweils unabhängig voneinander H, Halogen, -($C_1$-$C_4$)Alkyl, Nitro oder Cyano,

$R^{14}$, $R^{15}$ unabhängig voneinander H, unsubstituiertes oder durch ($C_1$-$C_4$)Alkoxy, Triazolyl oder Imidazolyl

substituiertes $(C_1-C_6)$Alkyl, Cyclo$(C_3-C_6)$alkyl, $(C_3-C_6)$Alkenyl, Phenyl oder Benzyl; oder

$R^{14}$ und $R^{15}$ gemeinsam mit dem Kohlenstoffatom, an das sie gebunden sind, ein unsubstituiertes oder durch Methyl oder Halogen substituiertes Cyclo-$(C_5-C_7)$alkyl,

$R^{16}$ $(C_1-C_4)$Alkyl, Phenyl, $(C_1-C_6$-Alkyl)carbonyl, Benzyl, Benzoyl, Halogenbenzyl, Halogenbenzoyl oder Methylbenzoyl,

$R^{17}$ H, $(C_1-C_4)$Alkyl, Formyl, $(C_1-C_6$-Alkyl)carbonyl, Benzoyl, Halogenbenzoyl, Methylbenzoyl oder Trihalogenacetyl und

$R^{18}$ $(C_1-C_4)$Alkyl, Phenyl oder Methylphenyl bedeuten,

sowie deren für landwirtschaftliche Zwecke verträglichen Salze und Quaternisierungsprodukte.

2. Verfahren zur Herstellung von Verbindungen der Formel I sowie ihrer Salze und Quaternisierungsprodukte, dadurch gekennzeichnet, daß man

a) einen Bisformylester der Formeln II a oder IIb

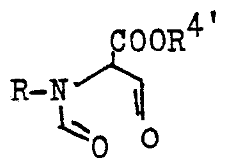

II a

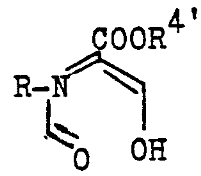

II b

wobei $R^{4'}$ = $(C_1-C_{12})$Alkyl bedeutet,

$(a_1)$ mit einem $(C_1-C_3)$Carbonsäureamid cyclisiert oder

$(a_2)$ mit Kaliumrhodanid zu einem 2-Mercaptoimidazol-Derivat umsetzt und dieses mit Salpetersäure und Natriumnitrit entschwefelt, oder

b) ein Phenylimidazolderivat der Formel III

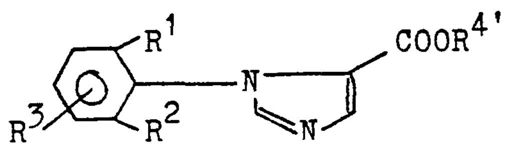

III

partiell oder vollständig im Phenylring hydriert und die unter a) oder b) erhaltenen Verbindungen derivatisiert.

3. Pflanzenschutzmittel mit einem wirksamen Gehalt an einer Verbindung der allgemeinen Formel (I) von Anspruch 1.

4. Verwendung von Verbindungen der allgemeinen Formel (I) von Anspruch 1 als Pflanzenschutzmittel.

5. Verfahren zur Wachstumsregulierung von Pflanzen, zur Bekämpfung von Schadpflanzen und Schadpilzen, dadurch gekennzeichnet, daß man auf die Pflanzen oder die Anbaufläche eine wirksame Menge einer Verbindung der allgemeinen Formel (I) von Anspruch 1 appliziert.

**Patentansprüche Österreich:**

1. Verfahren zur Herstellung von Verbindungen der Formel I,

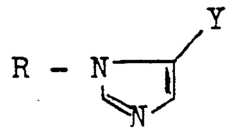 (I),

(I), worin

R = einen Rest der Formeln

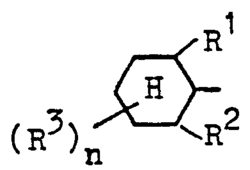 oder 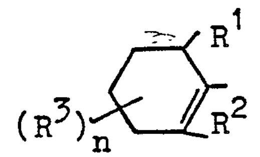 ,

n = 0 bis 6

Y = $-CN-$ $-COOR^4$, $-COSR^5$, 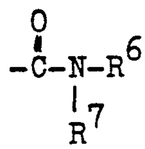, 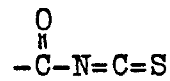

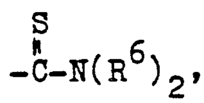, 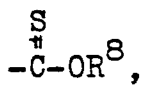, 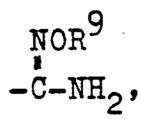, 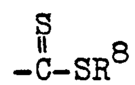, $-CH_2-OR^{10}$,

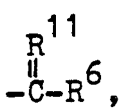, 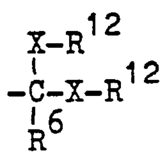, 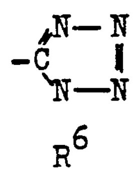 , 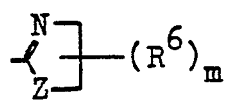,

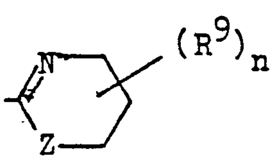 , 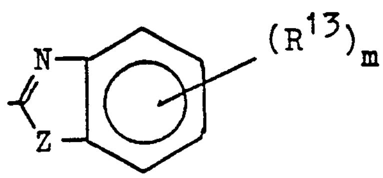 ,

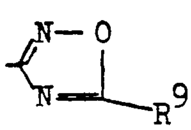 oder 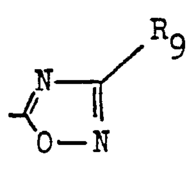 ,

m = 1 bis 4,

Z = O, S oder N-$R^6$,

X = O oder S,

$R^1$, $R^2$ unabhängig voneinander ($C_1$-$C_4$)Alkyl oder Wasserstoff,

$R^3$ jeweils unabhängig voneinander ($C_1$-$C_4$)Alkyl, -($C_1$-$C_4$)Alkoxy, ($C_1$-$C_4$)Hydroxyalkyl,

($C_1$-$C_4$)-Alkoxy($C_1$-$C_4$)alkyl, oder Halogen,

$R^4$ Wasserstoff, ($C_1$-$C_{12}$)Alkyl, ($C_1$-$C_{12}$)-Alkyl, das ein- bis dreifach substituiert ist Hydroxy, Halogen, Cyano, ($C_1$-$C_6$)Alkoxy, ($C_1$-$C_4$)Alkoxy($C_1$-$C_4$)alkoxy, -($C_1$-$C_4$)Alkylthio, ($C_1$-$C_4$)Alkylsulfinyl, ($C_1$-$C_4$)-Alkylsulfonyl, Mono-oder Di-($C_1$-$C_4$-alkyl)amino, Cyano, Aminocarbonyl, ($C_1$-$C_4$)Alkanoyl, ($C_1$-$C_4$-Alkoxy)carbonyl, Cyclo($C_3$-$C_7$)alkyl, Tri($C_1$-$C_4$)alkyl)-silyl, Benzyloxy, Benzyloxyethoxy, Phenyl oder Phenyl, das durch Halogen oder ($C_1$-$C_4$)Alkyl ein- oder mehrfach substituiert ist, oder Phenoxy, Phenylthio, die beide durch Halogen oder ($C_1$-$C_4$)-Alkyl ein-oder mehrfach substituiert sein können, ferner durch Oxiranyl, Tetrahydrofuryl, Triazolyl, Pyridinyl, Imidazolyl, Carboxy, Carboxylat mit einem für die Landwirtschaft einsetzbaren Kation oder den Rest -O-N = C($CH_3$)$_2$;

($C_3$-$C_6$)Alkenyl, halogeniertes ($C_3$-$C_6$)Alkenyl, unsubstituiertes oder durch Halogen oder ($C_1$-$C_4$)Alkyl substituiertes Cyclo($C_3$-$C_7$)alkyl, unsubstituiertes oder durch Halogen oder ($C_1$-$C_4$)Alkyl substituiertes Cyclo($C_5$-$C_7$)alkenyl, ($C_3$-$C_6$)Alkinyl, 1,2-Epoxy-prop-3-yl, Phenyl oder Phenyl, das ein oder zweifach durch Halogen, Nitro, Cyano, ($C_1$-$C_4$)Alkyl, ($C_1$-$C_4$-Alkoxy)carbonyl oder ($C_1$-$C_4$)Alkoxy substituiert ist.

($C_1$-$C_4$-Alkoxy)carbonyl, Phenylcarbonyl, wobei der Phenylring durch Halogen, Nitro, Cyano, ($C_1$-$C_4$)-Alkyl substituiert sein kann,

einen Rest der Formeln 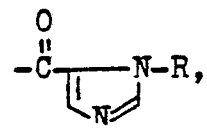

$-N{=}CR^{14}R^{15}$, $-NR^6R^{16}$,

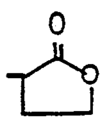, 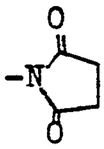, 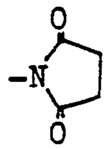 oder 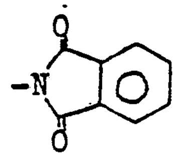 ;

$R^5$ ($C_1$-$C_{12}$)-Alkyl oder ($C_1$-$C_{12}$)Alkyl, das bis zu zweifach durch ($C_1$-$C_4$)Alkoxyethoxy, Cyclo($C_3$-$C_6$)alkyl, Benzyloxy, Phenyl, Phenoxy, ($C_1$-$C_4$)Alkylthio, ($C_1$-$C_4$-Alkoxy)carbonyl, Carboxy oder Carboxylat mit einem für die Landwirtschaft einsetzbares Kation, substituiert ist,

$R^6$ jeweils unabhängig voneinander Wasserstoff, ( $C_1$-$C_6$)Alkyl, Phenyl oder ($C_3$-$C_6$_-Alkenyl,

$R^7$ Wasserstoff, ($C_1$-$C_{12}$)Alkyl oder ($C_1$-$C_{12}$)Alkyl, das bis zu zweifach, durch ($C_1$-$C_{12}$)Alkyl oder ($C_1$-$C_4$)-Alkoxyethoxy, Hydroxy, Halogen, Cyclo($C_3$-$C_6$)alkyl, Benzyloxy, Cyano, Aminocarbonyl, Carboxy, ($C_1$-$C_4$-Alkoxy)carbonyl, Formyl, Phenyl oder Phenoxy substituiert ist, Phenyl oder Phenyl, das bis zu zweifach durch Halogen, Nitro, Cyano ($C_1$-$C_4$)Alkyl oder ($C_1$-$C_4$)Alkoxy substituiert ist; ($C_3$-$C_6$)-Alkenyl, -($C_3$-$C_6$))Cycloalkyl, einen Rest der Formeln $-NR^6R^{17}$, $-OR^9$, -NH-CO-$NH_2$, -NH-CS-$NH_2$ oder $-SO_2R^{18}$ oder

$R^6$ und $R^7$ bilden gemeinsam mit dem Stickstoffatom an das sie gebunden sind, einen gesättigten oder ungesättigten gegebenenfalls benzokondensierten drei-bis sieben gliedrigen Ring, der bis zu drei Heteroatome aus der Gruppe O, N und S enthält und der unsubstituiert oder durch ($C_1$-$C_4$)-Alkyl oder Halogen substituiert ist und eine Carbonylgruppe enthalten kann,

$R^8$ H, ($C_1$-$C_6$)Alkyl oder Phenyl, oder im Falle R = -CS-$OR^9$ ein für die Landwirtschaft einsetzbares Kation,

$R^9$ jeweils unabhängig voneinander H, ($C_1$-$C_4$)Alkyl oder Benzyl;

$R^{10}$ jeweils unabhängig voneinander H, unsubstituiertes oder durch Phenyl, Halogenphenyl, Nitrophenyl, Cyanophenyl, ($C_1$-$C_4$)Alkylphenyl oder ($C_1$-$C_4$)Alkoxyphenyl, Hydroxy, Cyano, ($C_1$-$C_4$-Alkoxy)-carbonyl, ($C_1$-$C_4$)Alkylthio, ($C_1$-$C_4$)Alkoxy, Cyclo($C_5$-$C_7$)alkyl oder Benzyloxy substituiertes ($C_1$-$C_{12}$)alkyl;

Cyclo($C_5$-$C_8$)alkyl, ($C_3$-$C_6$)Alkenyl, Halogen($C_3$-$C_6$)-alkenyl, ($C_3$-$C_6$)Alkinyl, Cyclo($C_5$-$C_6$)alkenyl, ($C_1$-$C_6$-Alkyl)carbonyl, Halogen($C_1$-$C_6$-alkyl)carbonyl, [($C_1$-$C_6$-Alkyl)amino]carbonyl, Benzoyl, Halogenbenzoyl oder Methylbenzoyl;

$R^{11}$ Sauerstoff (einschließlich der Bisufitaddukte) oder der Rest $=N-O-R^{10}$,

$R^{12}$ jeweils unabhängig voneinander unsubstituiertes oder durch Phenyl, Cyclo($C_5$-$C_7$)alkyl, ($C_1$-$C_4$)-Alkoxy, ($C_1$-$C_4$)Alkylthio oder Halogen substituiertes ($C_1$-$C_6$)Alkyl;

oder beide Rest $R^{12}$ gemeinsam mit X und dem Kohlenstoffatom an das sie gebunden sind, einen unsubstituierten oder durch ($C_1$-$C_4$)Alkyl, Hydroxy-($C_1$-$C_4$)alkyl, Halogen($C_1$-$C_4$)alkyl oder Phenyl substituierten 5-oder 6-gliedrigen gesättigten heterocyclischen Ring,

$R^{13}$ jeweils unabhängig voneinander H, Halogen, -($C_1$-$C_4$)Alkyl, Nitro oder Cyano,

$R^{14}$, $R^{15}$ unabhängig voneinander H, unsubstituiertes oder durch ($C_1$-$C_4$)Alkoxy, Triazolyl oder Imidazolyl substituiertes ($C_1$-$C_6$)Alkyl, Cyclo($C_3$-$C_6$)alkyl, ($C_3$-$C_6$)Alkenyl, Phenyl oder Benzyl; oder

$R^{14}$ und $R^{15}$ gemeinsam mit dem Kohlenstoffatom, an das sie gebunden sind, ein unsubstituiertes oder durch Methyl oder Halogen substituiertes Cyclo-($C_5$-$C_7$)alkyl,

$R^{16}$ ($C_1$-$C_4$)Alkyl, Phenyl, ($C_1$-$C_6$-Alkyl)carbonyl, Benzyl, Benzoyl, Halogenbenzyl, Halogenbenzoyl oder Methylbenzoyl,

$R^{17}$ H, ($C_1$-$C_4$)Alkyl, Formyl, ($C_1$-$C_6$-Alkyl)carbonyl, Benzoyl, Halogenbenzoyl, Methylbenzoyl oder Trihalogenacetyl und

$R^{18}$ ($C_1$-$C_4$)Alkyl, Phenyl oder Methylphenyl bedeuten,

sowie deren für landwirtschaftliche Zwecke verträglichen Salze und Quaternisierungsprodukte, dadurch gekennzeichnet, daß man,

a) einen Bisformylester der Formeln II a oder II b

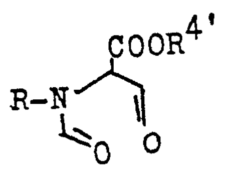

II a

⇌

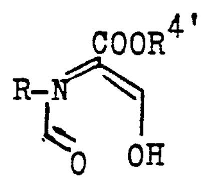

II b

wobei $R^{4'}$ = ($C_1$-$C_{12}$)Alkyl bedeutet,

($a_1$) mit einem ($C_1$-$C_3$)Carbonsäureamid cyclisiert oder

($a_2$) mit Kaliumrhodanid zu einem 2-Mercaptoimidazol-Derivat umsetzt und dieses mit Salpetersäure und Natriumnitrit entschwefelt, oder

b) ein Phenylimidazolderivat der Formel III

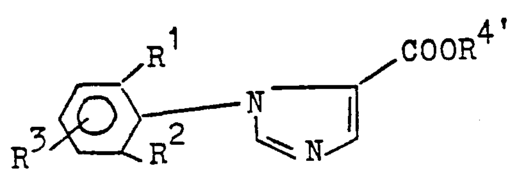

III

partiell oder vollständig im Phenylring hydriert und die unter a) oder b) erhaltenen Verbindungen derivatisiert.

2. Pflanzenschutzmittel mit einem wirksamen Gehalt an einer Verbindung der allgemeinen Formel (I) von Anspruch 1.

3. Verwendung von Verbindungen der allgemeinen Formel (I) von Anspruch 1 als Pflanzenschutzmittel.

4. Verfahren zur Wachstumregulierung von Pflanzen, zur Bekämpfung von Schadpflanzen und Schadpilzen, dadurch gekennzeichnet, daß man auf die Pflanzen oder die Anbaufläche eine wirksame Menge einer Verbindung der allgemeinen Formel (I) von Anspruch 1 appliziert.

5. Verfahren zur Bekämpfung von unerwünschtem Pflanzenwuchs, dadurch gekennzeichnet, daß man auf die Pflanzen oder die Anbaufläche eine wirksame Menge einer Verbindung der allgemeinen Formel (I) von Anspruch 1 appliziert.

6. Verfahren zur Bekämpfung von Schadpilzen, dadurch gekennzeichnet, daß man auf die Pflanzen oder die Anbaufläche eine wirksame Menge einer Verbindung der allgeemeinen Formel (I) von Anspruch 1 appliziert.